# EUROPEAN PATENT SPECIFICATION

(11) **EP 0 882 431 B1**
(45) Date of publication and mention of the grant of the patent: **09.10.2002**
(21) Application number: 98201852.5
(22) Date of filing: 04.06.1998
(51) Int. Cl.: A61B 17/72

(54) **Device for the elastic intramedullary synthesis of bone fractures**
Vorrichtung zur elastischen intramedullären Synthese von Knochenfracturen
Dispositif pour la synthèse intramédullaire élastique des fractures osseuses

(30) Priority: 05.06.1997 IT MI971322
(43) Date of publication of application: 09.12.1998
(73) Proprietor: Di Bartolomei, Claudio, 00123 Roma (IT); Gazzani, Romolo Igino, 15069 Serravalle Scrivia (Alessandria) (IT)
(72) Inventor: Di Bartolomei, Claudio, 00123 Roma (IT); Gazzani, Romolo Igino, 15069 Serravalle Scrivia (Alessandria) (IT)
(74) Representative: Martegani, Franco

(56) References cited:
- EP-A- 0 565 140
- DE-A- 4 210 801
- DE-A- 19 707 420
- FR-A- 2 727 304
- FR-A- 2 747 911
- PATENT ABSTRACTS OF JAPAN vol. 16, no. 133 (C-0925), 6 April 1992 & JP 03 295562 A (DAIDO STEEL), 26 December 1991

## Description

The present invention refers to a device for the elastic intramedullary synthesis of bone fractures, as for instance the femur, the humerus, the tibia, etc.

In the treatment of bone fractures, as for instance the femur, the humerus, the tibia, etc, it is currently known the insertion of pins and the like in order to reduce such fractures.

Further, suitably curved steel biocompatible wires, as AISI 316L, have been used, said wires can be elastically deformed and have rounded ends. These wires, at least two of them, are fixed, at one end, within a hollow member which has the role of an insertion element or funnel and they are held together, at the other end, by various holding and tie systems. This second link, positioned near the rounded ends, is then taken away, once the pin and the various wires have been inserted within the medullary canal. In such a way, the curved wires, which can be elastically deformed, go back to their original shape and push against the inner portion of the bone and engage with it.

Naturally, the various holding and linking systems of the free ends of the wires are a complication and they have to be removed in order that the wires have the bend or the curvature suitable to guarantee their requested final arrangement.

A device having the features of the first part of claim 1 is disclosed in FR-2.727.304.

A general purpose of the present invention is to solve the above mentioned technical problems relative to the known art in a very simple, economical and particularly functional way.

In consideration of the above purposes, according to the present invention, a device for the elastic intramedullary synthesis of bone fractures has been realised, said device having the features disclosed in the accompanying claims.

The structural and functional features and the advantages of the present invention in comparison with the known art will be clearer and more evident from the analysis of the following description, referred to the accompanying drawings, which show examples of embodiments according to the present invention. In said drawings:
- Figure 1 is a sectional schematic view of a femur with a meta-diaphysial fracture wherein a device according to the invention has been inserted;
- Figure 2 is a sectional schematic view of a femur with proximal fractures wherein a further embodiment of the device according to the invention has been inserted;
- Figure 3 is an enlarged elevation view of a sleeve used in Figure 1, 2 with an exploded view of its inner component parts;
- Figures 3a-3l show cross sectional view according to the corresponding lines S1-S10 as shown in Figure 3;
- Figure 4 is an enlarged elevation view of an adapter used in Figure 1 together with a sleeve, similar to the one of Figure 3, but with a changed end portion, as shown, and with its inner component parts also shown in an exploded view;
- Figures 4a-4e show cross sectional views according to the corresponding lines S11-S15 as shown in Figure 4;
- Figure 5 shows a sectional view of an accessory suitable to assembly and disassembly of the cap of the sleeve and to pull out both the sleeve and the adapter;
- Figure 6 is a sectional view of a further accessory to pull out the wires;
- Figure 7 shows a device according to the invention after being assembled within a bone;
- Figures 8 and 9 show different embodiments of the sleeve which can be used in the application of Figure 7.

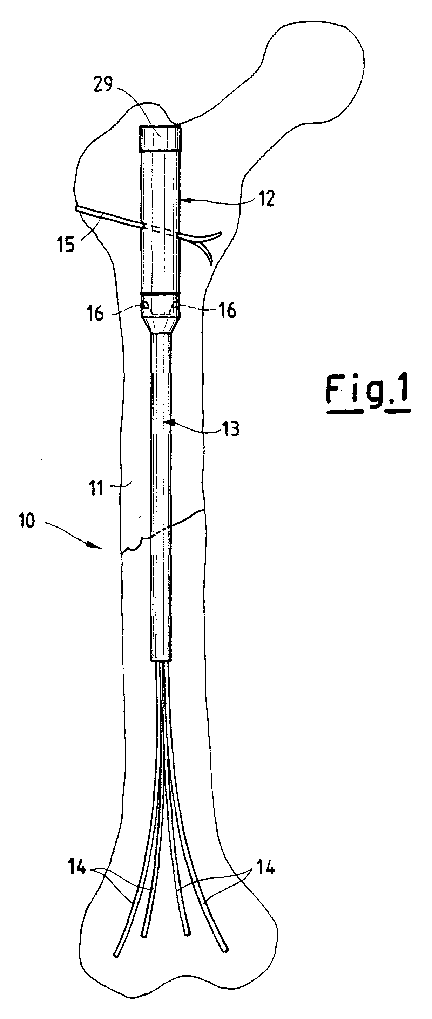

With reference to the drawings, numeral 10 indicates the device of the invention, said device, in the example of Figure 1, is applied to the meta-diaphysial fracture of a femur 11.

The device 10 comprises a sleeve 12, and adapter 13 and wires 14 and 15 according to the invention.

First of all, it is important to point out that, according to the invention, the wires 14 and 15 are metallic wires with programmable shape memory. These wires are made, for instance, of biocompatible titanium-nickel of various lengths and sections, and said wires can be cold deformed. When the wires are heated by the body temperature, they return to their originally given shape.

The sleeve 12 is internally hollow, with cavities having sections of various shapes and is made of titanium to guide the wires 14 and 15 and to insert said wires.

The adapter 13 is hollow and it is also made of titanium and it is engaged to the sleeve 12 by means of stop dowels and by similar stop elements 16.

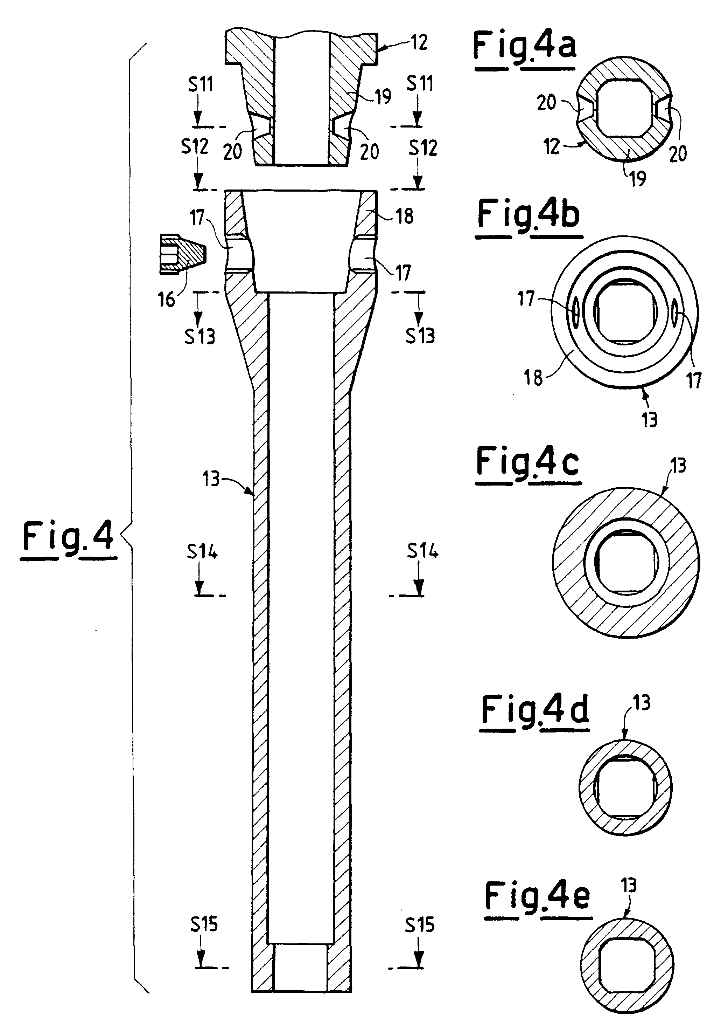

Figure 4 shows the presence of through bores 17 on the upper end 18 of the adapter 13.

This end 18 of the adapter 13 is enlarged, hollow and has a frusto conical shape and it is suitable to receive the lower end 19 of the sleeve 12, which has a complementary shape. The end 19 has countersunk bores 20 which can be aligned to the bores 17 to receive the stop dowels 16.

In the example of Figure 1, the sleeve 12 receives the four wires 14 which also extend substantially axially within the following adapter 13 and then they extend into the medullary canal within the bone 11.

Further, the sleeve 12 receives an additional wire 15 which is transversely inserted in the proximal cortex, in order to improve the steady positioning of the entire device.

The sleeve 12, in a hollow portion 21 above the passage area of said wire 15 inside a corresponding bore 22, houses an insert 23 which has through bores 24 for the wires 14. The insert 23 has a hexagonal shape as well as the hollow portion 21 in order to avoid the rotation of the wires 14. The ends of the wires 14 are provided with a washer 25, provided with bores 26 to house said ends of the wires 14. The washer has a circular shape so that it can be inserted in a further hollow portion 27 which has a circular section and is obtained inside the sleeve, above the hollow portion 21.

A cap or cover 28, also made of titanium, is externally threaded and is the closing element of the internally threaded upper hollow portion 27. In this way, the cap 28 locks the wires and receives a further cap or lockup 29, made, for instance, of Teflon.

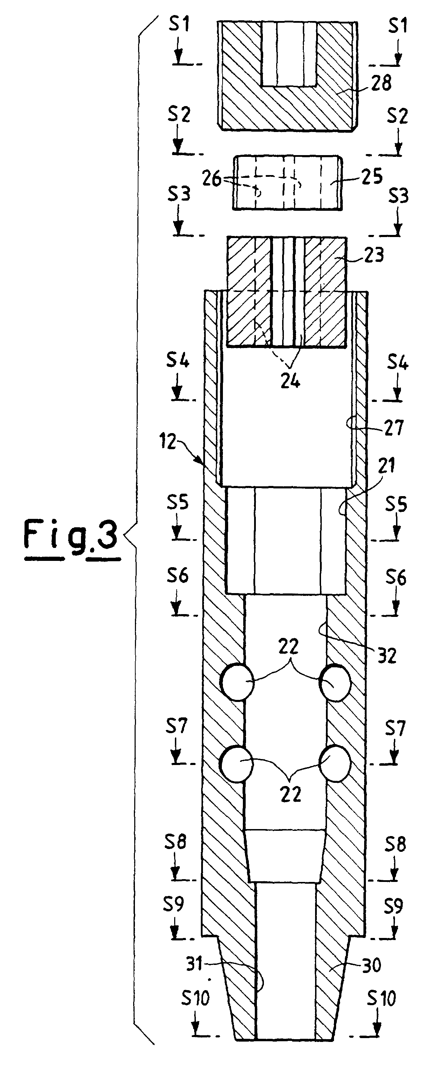

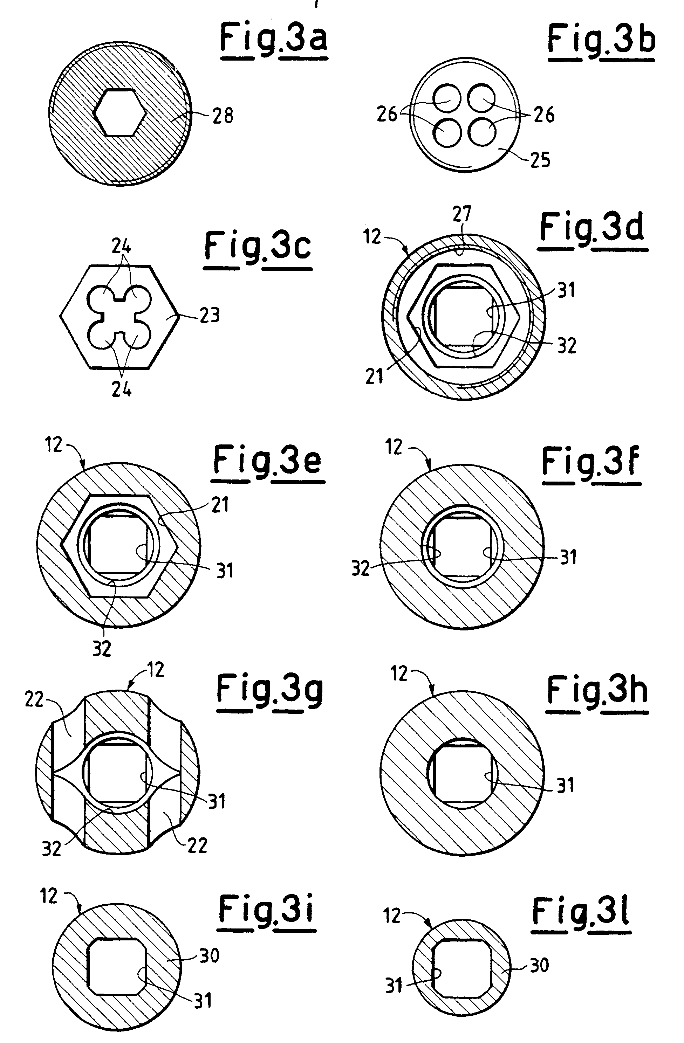

Figure 3 shows in more details where the positioning of the components can be realised, and sections 3a-31 show the various shapes of both the hollow of the sleeve and of the elements contained therein.

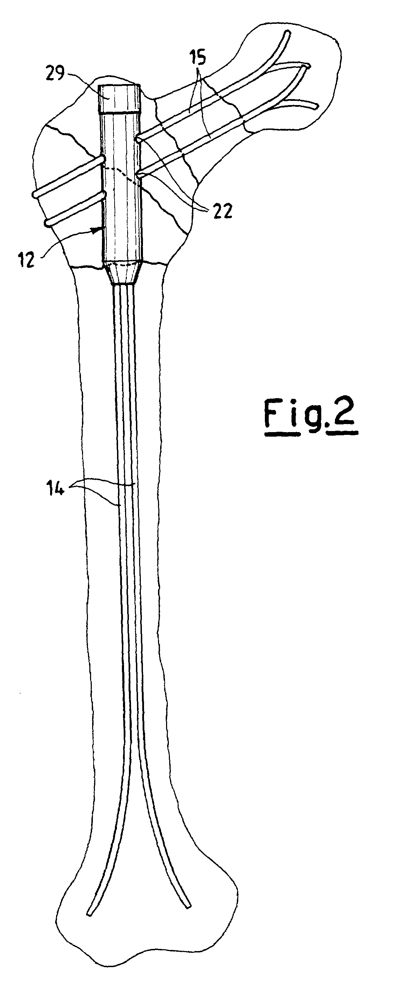

The sleeve 12 has a lower end portion 30 which has a reduced section of its inner square cavity 31, to avoid the rotation of the wires 14. A further portion with a circular section 32 is the intermediate section wherein the bores 22 for the transverse and variously inclined wires 15, as the ones shown in Figures 1 and 2, are obtained.

Figure 2 shows a femur with proximal fractures wherein a sleeve 12 has simply been inserted. The sleeve 12 is substantially the one shown in details in Figure 3 which has been previously described in details.

In this case, it is interesting to notice that the couples of wires 15, which are inserted inside the bores 22, have the same behaviour of the wires 14.

In case of femur proximal fractures associated to other bifocal meta-diaphysial fractures, both the embodiments of Figure 1 and Figure 2 are utilised.

The device is suitable to treat the fractures of the tibia, humerus and the supra- and dia-condylar fractures of the femur.

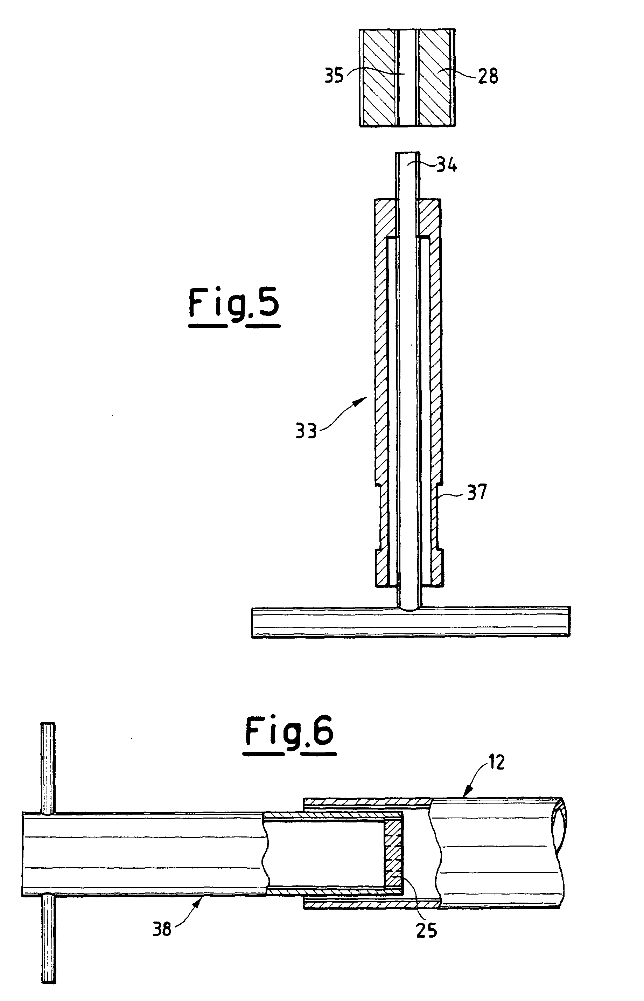

Figure 5 shows a sectional view of an accessory, indicated by numeral 33, which is conveniently used in the various operating phases of the device of the invention.

The accessory 33 comprises a central screw 34, to screw the cap 28 inside the hollow portion 27. The central screw 34 drives a seat 35 of the cap 28, said screw is driven by a specific locking sleeve 36 positioned around the screw and said sleeve can be driven by inserting a key in a seat 37 formed on said sleeve.

Using this accessory it is possible both to assemble and to disassemble the cap 28, while the final pull out of the sleeve 12 and of the adapter 13 can be executed through another threaded engagement by striking hammer.

Figure 6 shows another accessory, indicated by numeral 38, said accessory is used as a wrench for the washer 25, so as to pull out the wires before the sleeve and the adapter are pulled out.

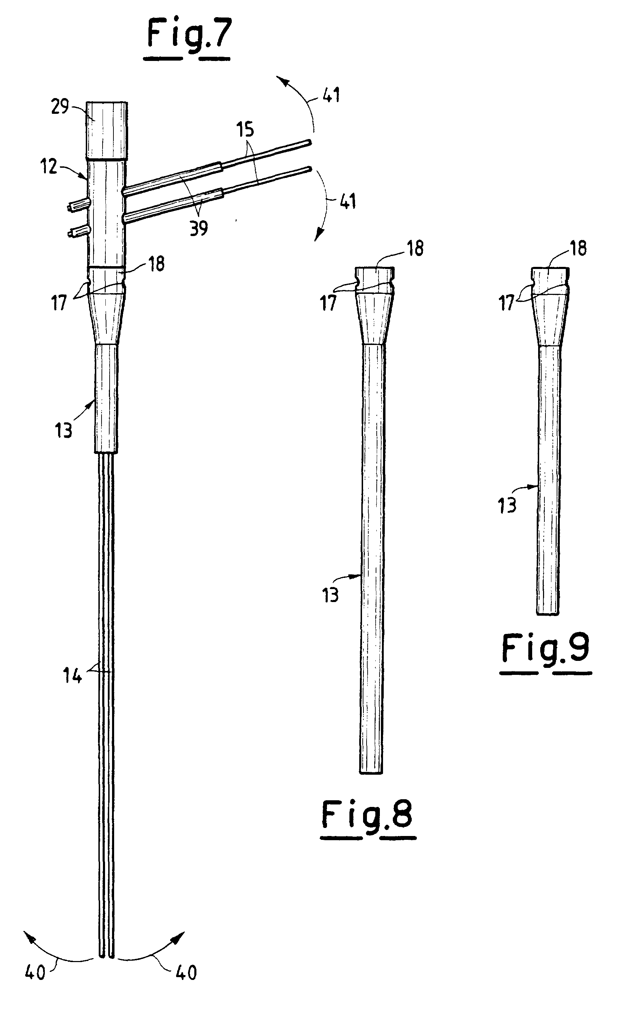

Finally, Figure 7 shows the device of the invention assembled within the bone.

The wires 14 are perfectly straight when they are inserted in the sleeve and in the adapter. Then, once positioned inside the bone, said wires start buckling, due to the body temperature, along the arrows 40 of Figure 7 until they return to their original shape and get engaged to the bone.

The original memorised shape of the nickel titanium wires advantageously allows to program the degree of expansion and consequently the intramedullary pressure exercised on the spongy inner cortical surfaces of the bone.

The sleeve 12 can have various dimensions as the ones shown in the following Figures 8 and 9, according to the fracture level in the diaphysial area to go beyond said fractures.

The use of sleeves with various lengths and diameters allows to go beyond the fracture area, for instance in the case of multiple seriously compound fractures, bifocal fractures, etc. In this way, the possible serious complication of having the metallic wires coming out from the medullary canal during the insertion phase is avoided.

Further, Figure 7 shows how the wires 15 can be inserted inside the bores 22 by using additional small sleeves 39 made of titanium, wherein the previously mentioned wires 15 slide.

Also the wires 15, provided with sleeves 39, are straight during the insertion phase and then, once positioned within the bone, due to the body temperature, they start buckling along the arrows 41 of Figure 7. In this way the wires return to their original shape and get engaged to the bone.

With reference to the various figures and embodiments and to the previous description, it is evident that the device of the present invention overcomes the problems related to the known art.

In fact, this device guarantees a very easy implantation in a wide range of clinical uses. Further, the precise positioning of the wires provided with memory is realised both during the insertion phase and during the programmed opening based on the temperature.

With such a device, an excellent restoration of the fractured areas is obtained and said device can be pulled out very easily once the fracture has healed.

The purpose set forth in the preamble of the description is then achieved.

Naturally, the embodiments of the device can be of any shape and, at any rate, different from the ones shown in the drawings by way of non limiting examples.

The protection of the invention is therefore defined by the accompanying claims.

## Claims

1. A device for the elastic intramedullary synthesis of bone (11) fractures, comprising at least two wires, made of biocompatible metallic material, which can be inserted inside the bone in order to be fixed to the intramedullary cortex, said wires, when inserted in the medullary canal, being in their straight position, said wires (14, 15) being made of metallic material provided with a programmable shape memory and when heated by the body temperature, return to their originally given shape,
**characterised in that** said wires (14) are inserted inside a sleeve. (12) provided with an inner cavity (21, 27, 32, 31) which has variable cross sections and **in that** said wires are guided by elements (23, 25) which do not allow the rotation of said wires.

2. A device as claimed in claim 1, **characterised in that** said wires (14) go through the bores (26) of a washer (25) which can be positioned on an insert (23) which opposes the rotation movement, said insert is also provided with through bores (24) for said wires.

3. A device as claimed in claim 1, **characterised in that**, as a closing means for said inner cavity (21, 27, 32, 31), a cap (28) externally threaded is placed on an internally threaded portion (27) of said cavity.

4. A device as claimed in claim 1, **characterised in that** said sleeve (12) can be connected, at one of its ends (19), to an adapter (13), which is internally hollow to allow the passage of said wires (14).

5. A device as claimed in claim 4, **characterised in that** said end (19) of said sleeve (12) is suitable to be inserted inside a hollow end (18) of said adapter (13), wherein locking elements (16) are provided between said sleeve (12) and said adapter (13).

6. A device as claimed in claim 1, **characterised in that** said sleeve (12) is provided with bores (22) which are transversely positioned to receive additional wires (15).

7. A device as claimed in claim 6, **characterised in that** said additional wires (15) can slide inside small additional sleeves (39) placed inside said bores (22).

## Patentansprüche

1. Vorrichtung zur elastischen intramedullären Synthese von Frakturen von Knochen (11) mit zumindest zwei aus einem biologisch verträglichen metallischen Material gefertigten Drähten, die in den Knochen eingesetzt werden können, um am intramedullären Kortex fixiert zu werden, wobei sich die Drähte, wenn sie in den intramedullären Kanal eingesetzt werden, in ihrer geraden Position befinden, und wobei die Drähte (14, 15) aus einem metallischen Material mit einem programmierbaren Formgedächtnis gefertigt sind und bei Erwärmung auf Körpertemperatur zu ihrer ihnen ursprünglich verliehenen Form zurückkehren,
**dadurch gekennzeichnet, dass** die Drähte (14) in eine Muffe (12) eingesetzt sind, die einen Innenhohlraum (21, 27, 32, 31) aufweist, der veränderliche Querschnitte hat und in dem die Drähte durch Elemente (23, 25) geführt sind, die keine Drehung der Drähte zulassen.

2. Vorrichtung nach Anspruch 1,
**dadurch gekennzeichnet, dass** sich die Drähte (14) durch die Bohrungen (26) einer Scheibe (25) erstrecken, die auf einem Einsatz (23) positioniert werden kann, welcher der Drehbewegung entgegenwirkt, wobei der Einsatz ebenfalls mit Durchgangsbohrungen (24) für die Drähte versehen ist.

3. Vorrichtung nach Anspruch 1,
**dadurch gekennzeichnet, dass** als Schließeinrichtung für den Innenhohlraum (21, 27, 32, 31) eine Kappe (28) mit einem Außengewinde auf einem mit einem Innengewinde versehenen Abschnitt (27) des Hohlraums angeordnet wird.

4. Vorrichtung nach Anspruch 1,
**dadurch gekennzeichnet, dass** die Muffe (12) an einem ihrer Enden (19) mit einem Adapter (13) verbunden werden kann, der innen hohl ist, um den Durchtritt der Drähte (14) zu ermöglichen.

5. Vorrichtung nach Anspruch 4,
**dadurch gekennzeichnet, dass** das Ende (19) der Muffe (12) dafür geeignet ist, in ein hohles Ende (18) des Adapters (13) eingesetzt zu werden, wobei Sperrelemente (16) zwischen der Muffe (12) und dem Adapter (13) bereitgestellt sind.

6. Vorrichtung nach Anspruch 1,
**dadurch gekennzeichnet, dass** die Muffe (12) mit quer angeordneten Bohrungen (22) versehen ist, um zusätzliche Drähte (15) aufzunehmen.

7. Vorrichtung nach Anspruch 6,
**dadurch gekennzeichnet, dass** die zusätzlichen Drähte (15) in kleine zusätzliche Muffen (39) gleiten können, die in den Bohrungen (22) angeordnet sind.

## Revendications

1. Dispositif pour la synthèse médullaire élastique de fractures d'os (11), comprenant au moins deux câbles en matériau métallique biocompatible, pouvant être insérés dans l'os pour être fixés au cortex médullaire, lesdits câbles, une fois insérés dans le canal médullaire, étant dans une position rectiligne, lesdits câbles (14, 15) étant en matériau métallique à mémoire de forme programmable, et retrouvant leur forme initiale lorsqu'ils sont chauffés par la température du corps, **caractérisé en ce que** lesdits câbles (14) sont insérés dans un manchon (12) muni d'une cavité intérieure (21, 27, 32, 31) présentant un profil variable en coupe transversale, et **en ce que** les câbles sont guidés par des éléments (23, 25) interdisant une rotation desdits câbles.

2. Dispositif selon la revendication 1, **caractérisé en ce que** lesdits câbles (14) traversent des trous (26) d'une rondelle (25) pouvant être disposée sur un insert (23) s'opposant au mouvement de rotation, ledit insert étant également muni de trous (24) pour le passage des câbles.

3. Dispositif selon la revendication 1, **caractérisé en ce que**, comme moyen de fermeture de ladite cavité intérieure (21, 27, 32, 31), un bouchon (28) fileté extérieurement est placé sur une portion filetée intérieurement de ladite cavité (21, 27, 32, 31).

4. Dispositif selon la revendication 1, **caractérisé en ce que** le manchon (12) peut être relié à une de ses extrémités (19) à un adaptateur (13) qui est creux intérieurement pour permettre le passage desdits câbles (14).

5. Dispositif selon la revendication 4, **caractérisé en ce que** ladite extrémité (19) dudit manchon (12) est prévue pour être insérée dans une extrémité creuse (18) de l'adaptateur (13), dans laquelle des éléments de verrouillage (16) sont prévus entre ledit manchon (12) et ledit adaptateur (13).

6. Dispositif selon la revendication 1, **caractérisé en ce que** ledit manchon (12) est muni de trous (22) disposés transversalement pour recevoir des câbles supplémentaires (15).

7. Dispositif selon la revendication 6, **caractérisé en ce que** lesdits câbles supplémentaires (15) peuvent coulisser dans des petits manchons supplémentaires (39) placé dans lesdits trous (22).
